(19) Europäisches Patentamt
European Patent Office
Office européen des brevets

(11) **EP 3 145 412 B1**

(12) **EUROPÄISCHE PATENTSCHRIFT**

(45) Veröffentlichungstag und Bekanntmachung des Hinweises auf die Patenterteilung:
**26.06.2024   Patentblatt 2024/26**

(21) Anmeldenummer: **15723027.7**

(22) Anmeldetag: **19.05.2015**

(51) Internationale Patentklassifikation (IPC):
**A61B 8/06** *(2006.01)*      **A61B 5/1455** *(2006.01)*

(52) Gemeinsame Patentklassifikation (CPC):
**A61B 5/0048; A61B 5/1455; A61B 8/085; A61B 8/488; G01N 21/4795;** A61B 5/0097; A61B 5/01; A61B 5/14532; A61B 5/14552; A61B 5/7228; A61B 8/4416; A61B 2562/0233; G01N 2021/4709

(86) Internationale Anmeldenummer:
**PCT/EP2015/061005**

(87) Internationale Veröffentlichungsnummer:
**WO 2015/177156 (26.11.2015 Gazette 2015/47)**

(54) **VERFAHREN ZUR NICHTINVASIVEN OPTISCHEN MESSUNG VON EIGENSCHAFTEN VON FLIESSENDEM BLUT**

METHOD FOR NON-INVASIVE OPTICAL MEASUREMENT OF PROPERTIES OF FREE-FLOWING BLOOD

PROCÉDÉ DE MESURE OPTIQUE NON INVASIVE DE PROPRIÉTÉS DE SANG EN CIRCULATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.05.2014   DE 102014107261**

(43) Veröffentlichungstag der Anmeldung:
**29.03.2017   Patentblatt 2017/13**

(73) Patentinhaber: **NIRLUS Engineering AG 23560 Lübeck (DE)**

(72) Erfinder: **HERRMANN, Vera 23560 Lübeck (DE)**

(74) Vertreter: **von dem Borne, Andreas et al Andrejewski - Honke Patent- und Rechtsanwälte Partnerschaft mbB An der Reichsbank 8 45127 Essen (DE)**

(56) Entgegenhaltungen:
**EP-A1- 1 601 285     US-A1- 2006 253 007**

## Beschreibung

Die Erfindung betrifft ein Verfahren zur nichtinvasiven optischen in-vivo-Messung von Eigenschaften von fließendem Blut in einem Blutgefäß im Innern eines Körpers. Messung von Eigenschaften von fließendem Blut meint z. B. die Bestimmung der Konzentration von Blutbestandteilen, z. B. die Glukosekonzentration, die Hämoglobinkonzentration oder auch die Sauerstoffsättigung des Blutes. Das erfindungsgemäße Verfahren betrifft aber auch die Messung der Temperatur des fließenden Blutes im Innern des Körpers. Im Vordergrund der Erfindung steht dabei die optische Analyse mittels Licht, z. B. Laserstrahlung durch Auswertung des rückgestreuten Lichtes, wobei der Ort der Messung, nämlich die Blutbahn, mittels gepulster Ultraschallstrahlung "markiert" wird. Dabei wird Licht beispielsweise einer Laserlichtquelle in den Körper eingestrahlt und durch Messung und Auswertung des rückgestreuten Streulichtes werden die gesuchten Parameter auf verschiedenste Weise bestimmt. Dabei wird üblicherweise elektromagnetische Strahlung, z. B. Laserstrahlung, aus dem sichtbaren Bereich und dem Infrarotbereich verwendet, da lebendes Gewebe für elektromagnetische Strahlung zwischen etwa 550 nm und 1000 nm weitgehend transparent ist (biologisches Fenster). Die Lokalisierung des Messortes mittels Ultraschallstrahlung basiert auf der Wechselwirkung des Ultraschallwellenfeldes mit dem Blut bzw. Gewebe. Das Ultraschallwellenfeld verursacht durch Wechselwirkung mit Blut und Gewebe Änderungen der optischen Eigenschaften, insbesondere des Reflexionsbzw. Streuvermögens. Dieses führt zu einer Modulation des rückgestreuten Lichtes mit der Frequenz der Ultraschallstrahlung, so dass sich im Zuge der Auswertung der modulierte Anteil extrahieren lässt.

Ein solches Verfahren zur optischen Messung von Eigenschaften von fließendem Blut mit Ultraschalllokalisierung ist z. B. aus der EP 1 601 285 B1 bekannt. Die Ultraschallstrahlung wird auf das Innere eines zentralen Blutgefäßes fokussiert und eine feste Pulslänge und Repititionszeit für die Ultraschallstrahlung wird vorgegeben. Außerdem werden eine Lichtquelle sowie eine benachbarte Detektionseinheit zum Erfassen des rückgestreuten Lichtes auf der Hautoberfläche über dem Blutgefäß derart positioniert, dass der Abstand zwischen Lichtquelle und der Mehrheit der Lichtrezeptoren der Detektionseinheit mit der Tiefe des untersuchten Blutgewebes korrespondiert. Das Zielgewebe wird mit wenigstens zwei diskreten Lichtwellenlängen beleuchtet und das rückgestreute Licht wird gemessen und über die Detektorfläche und eine Vielzahl von Ultraschallpulsen integriert. Aus den ermittelten Werten lässt die Konzentration im Blutgefäß unter Berücksichtigung des zum Signal beitragenden Volumens des Ultraschallfokus und der Blutgeschwindigkeit berechnen. Wesentlich ist dabei die Fokussierung des Ultraschallfeldes auf den Ort der Messung, nämlich auf die Blutbahn, da auf diese Weise die Quellenlokalisation realisiert wird.

In der DE 10 2006 036 920 B3 wird ein Verfahren zur Messung der Glukosekonzentration in pulsierendem Blut beschrieben, wobei innerhalb eines Messzyklus das Transmissions- und/oder Streuvermögen des Blutes durch wenigstens zwei eingestrahlte NIR-Wellenlängen mehrfach erfasst und ein von der Blutglukosekonzentration abhängiger Indikatorwert berechnet und die Blutglukosekonzentration durch Vergleich des Indikatorwertes mit einer zuvor bestimmten Kalibriertabelle ermittelt wird. Dabei wird die erste Wellenlänge aus dem Wellenlängenbereich 1560 bis 1630 nm ausgewählt und die zweite Wellenlänge wird aus dem Wellenlängenbereich 790 bis 815 nm ausgewählt und das Verhältnis der Transmissions- und/oder Streuvermögen der beiden Wellenlängen wird berechnet, wobei dieses Verhältnis in Relation zur Bluttemperatur als Indikatorwert zum Ablesen der Blutglukosekonzentration aus der Kalibriertabelle dient. Wesentlich ist dabei eine möglichst exakte Bestimmung der Bluttemperatur.

Vor diesem Hintergrund beschreibt die DE 10 2008 006 245 A1 ein Verfahren zur nichtinvasiven, optischen Bestimmung der Temperatur eines Mediums, vorzugsweise eines wasserhaltigen Mediums, wobei das zu untersuchende Medium mit infrarotem und/oder sichtbarem Licht im Bereich einer Absorptionslinie beleuchtet wird, deren Lage von der Temperatur des Mediums abhängt, und wobei die Absorption des Lichtes im Bereich der Absorptionslinie gemessen und aus dieser Messung durch Vergleich mit Kalibrierungsdaten die Temperatur ermittelt wird. Wesentlich ist dabei, dass das Medium mit zumindest zwei diskreten Lichtwellenlängen beleuchtet wird, welche im Bereich der Absorptionslinie auf unterschiedlichen Seiten des Absorptionsmaximums liegen und dass aus dem Verhältnis bzw. einem funktionellen Zusammenhang dieser beiden ermittelten Absorptionswerte zueinander zumindest ein von der Temperatur abhängiger Messwert oder eine von der Temperatur abhängige Messfunktion bestimmt wird und dass aus diesem Messwert bzw. dieser Messfunktion durch Vergleich mit den zuvor aufgenommenen Kalibrierungsdaten die Temperatur bestimmt wird. Auch bei dieser optischen Temperaturmessung kann der Ort der Messung im Innern eines Körpers, z. B. eine Blutbahn, mittels gepulster Ultraschallstrahlung markiert werden.

Das Prinzip des "Ultraschall-Tagging" hat sich bei der nichtinvasiven optischen Messung von Eigenschaften von fließendem Blut grundsätzlich bewährt. Das Verfahren ist jedoch weiterentwicklungsfähig, um die Qualität der Messung zu optimieren.

Im Übrigen wird in der US 2006/0253007 A1 ein medizinisches Diagnosesystem beschrieben, mit dem eine nicht-invasive oder minimalinvasive Detektion von Eigenschaften des Blutes möglich ist, wobei auch hier mit einer Kombination aus Ultraschallstrahlung und optischer Strahlung gearbeitet wird.

Der Erfindung liegt die Aufgabe zugrunde, ein Verfahren zu schaffen, welches eine verbesserte in-vivo-Messung von Eigenschaften von fließendem Blut in einem Blutgefäß im Innern eines Körpers ermöglicht.

Zur Lösung dieser Aufgabe lehrt die Erfindung ein Verfahren zur nichtinvasiven optischen in-vivo-Messung von Eigenschaften von fließendem Blut in einem Blutgefäß im Innern eines Körpers, z. B. zur Bestimmung der Konzentration von Blutbestandteilen, mit den Merkmalen des Anspruchs 1,

> wobei der Körper zur Markierung eines Blutgefäßes mit nicht fokussierter Ultraschallstrahlung mit einer Ultraschallfrequenz (fus) bestrahlt wird,

> wobei der Körper mit dem Blutgefäß mit Licht mit zumindest einer Lichtwellenlänge beleuchtet und das rückgestreute Licht mit einem Detektor erfasst wird,

> wobei der außerhalb des Blutgefäßes aus dem Körper rückgestreute Lichtanteil mit einer Frequenz $f_{MG}$ moduliert ist, welche der Frequenz fus der Ultraschallstrahlung entspricht,

> wobei der innerhalb des Blutgefäßes rückgestreute Lichtanteil aufgrund des Dopplereffektes mit fließendem Blut mit einer um die Dopplerverschiebung $f_D$ verschobenen Frequenz $f_{MB}$ moduliert ist und

> wobei mit einer Auswerteeinheit aus dem an dem Detektor gemessenen

Detektorsignal nur der mit der verschobenen Frequenz $f_{MB}$ modulierte Signalanteil extrahiert wird. - Aus diesem Signalanteil wird dann die Eigenschaft des Blutes, z. B. die Konzentration von Blutbestandteilen oder auch die Temperatur des Blutes, bestimmt.

Die Erfindung geht zunächst einmal von der bekannten Erkenntnis aus, dass sich Eigenschaften von fließendem Blut im Innern eines Körpers nicht invasiv und in-vivo mit optischen Methoden messen lassen, wenn zugleich eine Markierung des Messortes mittels Ultraschallstrahlung erfolgt. Beim vorbekannten Stand der Technik wurde im Zuge der Auswertung der gesamte Lichtanteil, der mit der Frequenz der Ultraschallstrahlung moduliert ist, extrahiert, und zwar unabhängig davon, ob das Licht tatsächlich aus der Blutbahn oder evtl. aus angrenzendem Gewebe rückgestreut wurde. Dieses ist beim Stand der Technik deshalb möglich, weil die Ultraschallstrahlung auf die Blutbahn fokussiert wird, so dass der modulierte Anteil des Lichtes, der außerhalb der Blutbahn rückgestreut wird, gering sein soll. Der Erfolg dieses Verfahrens hängt folglich in besonderem Maße von der Fokussierung der Ultraschallstrahlung ab, denn wenn mit Hilfe der Ultraschallstrahlung auch eine Modulation des Gewebes außerhalb der Blutbahn erfolgt, wird das Messsignal verfälscht.

Demgegenüber wird durch das erfindungsgemäße Verfahren gewährleistet, dass tatsächlich nur solche Lichtanteile des rückgestreuten Lichtes in die Auswertung einfließen, welche tatsächlich aus dem Blut rückgestreut werden. Dabei geht die Erfindung von der Erkenntnis aus, dass die aus dem fließenden Blut einerseits und dem umgebenden Gewebe andererseits rückgestreuten Lichtanteile mit unterschiedlichen Modulationsfrequenzen moduliert sind. In dem umgebenden Gewebe ist die Modulationsfrequenz $f_{MG}$ gleich der Ultraschallfrequenz fus. In fließendem Blut erfolgt jedoch aufgrund des Dopplereffektes eine Modulation mit einer veränderten Frequenz $f_{MB}$. Diese Modulationsfrequenz $f_{MB}$ unterscheidet sich von der Ultraschallfrequenz fus aufgrund der Bewegung des Blutes um die Frequenz der Dopplerverschiebung $f_D$:

$$f_{MB} = f_{US} \pm f_D.$$

Die Dopplerverschiebung $f_{Decho}$, die in einem Ultraschallecho registriert wird, ist wie folgt definiert:

$$f_{Decho} = (2 \, v_B \cdot f_{US} \cdot \cos \Phi)/_{US}$$

Die Dopplerverschiebung $f_D$, welche für die Veränderung der Modulationsfrequenz relevant ist, wird um das Zweifache kleiner als die Ultraschallecho-Doppler-Verschiebung ($F_{Decho} = 2 \cdot f_D$). Sie ergibt sich aus der folgenden Gleichung:

$$f_D = (v_B \cdot f_{US} \cdot \cos \Phi) \, /v_{US}$$

Dabei ist fus die Ultraschallfrequenz, $f_D$ die Dopplerverschiebung im Blut, $f_{Decho}$ die Dopplerverschiebung im Ultraschallempfänger, $v_B$ die Blutgeschwindigkeit, vus die Geschwindigkeit der Ultraschallwelle im Blut und $\Phi$ der Winkel zwischen der Richtung der Blutbewegung und der Ultraschallwelle.

Erfindungsgemäß gelingt folglich unter Ausnutzung des Dopplereffektes die präzise Ortung der Blutbahn, und zwar ohne dass mit fokussierter Ultraschallstrahlung gearbeitet wird. Der Dopplereffekt wurde zwar auch beim vorbekannten Stand der Technik verwendet, dort jedoch nur zur Vorbereitung der Messung, um den Ort der Messung überhaupt zu lokalisieren. Dieses ist auch im Rahmen der Erfindung in ähnlicher Weise möglich. Zum Auffinden des Blutgefäßes wird der gepulste Ultraschall in das Gewebe oberhalb des Blutgefäßes unter entsprechendem Winkel eingestrahlt und das Ultraschallecho ausgewertet. Durch Auswerten des Ultraschallechos lässt sich im Zuge des Abtastens der Tiefe die Blutbahn zunächst einmal auffinden und lokalisieren. Im Gegensatz zum Stand der Technik beschränkt sich die Erfindung jedoch nicht auf die Ausnutzung des Dopplereffektes im Zuge des Auffindens der Blutbahn, sondern der Einfluss der Dopplerverschiebung fließt auch in die Auswertung der optischen Messung ein. Denn im Zuge der Auswertung der optischen Messung werden nicht lediglich die mit der Frequenz der Ultraschallstrahlung modulierten Lichtanteile extrahiert, sondern nur exakt die mit der um die Dopplerverschiebung verschobenen Frequenz modulierten Lichtanteile, da nur diese auf eine Streuung innerhalb des bewegten Blutes zurückgehen. Damit erfolgt eine exakte Isolierung des Anteils des aus dem Blut rückgestreuten Photonenstroms.

Das rückgestreute Licht setzt sich folglich aus einem überhaupt nicht modulierten rückgestreuten Photonenstrom aus dem gesamten Gewebe, aus einem mit der Ultraschallfrequenz modulierten Photonenstrom aus dem mit der Ultraschallstrahlung beaufschlagten Gewebe und einem mit der verschobenen Frequenz modulierten Photonenstrom zusammen, der tatsächlich aus dem fließenden Blut rückgestreut wird. Durch eine geeignete Auswertung lässt sich dieser letzte Anteil aus dem Signal extrahieren und für die Bestimmung der gewünschten Eigenschaften des Blutes heranziehen.

Das erfindungsgemäße Verfahren zeichnet sich folglich durch ein sehr gutes Signal/Rauschverhältnis aus. Es gelingt eine exakte Isolierung des Lichtanteils der aus dem bewegten Blut zurückgestreut wird, ohne dass mit fokussierter Ultraschallstrahlung gearbeitet wird. Dieses gelingt auch weiterhin bei einfachem apparativem Aufbau. Wichtig ist die Tatsache, dass mit einem einfachen Detektor gearbeitet werden kann, da der Detektor - hinsichtlich der Lichtfrequenz - weder ortsaufgelöst noch frequenzaufgelöst messen muss. Mit dem Detektor werden lediglich Intensitäten und folglich lediglich ein Photonenstrom gemessen. Eine Analyse der Frequenz des rückgestreuten Lichts oder auch eine Phasenanalyse sind nicht erforderlich. Es erfolgt jedoch in der beschriebenen Weise eine Auswertung hinsichtlich der Frequenz, mit der das Licht aufgrund der Ultraschallstrahlung moduliert ist. Die Erfindung zeichnet sich folglich durch eine optimierte Quellenlokalisation und durch ein verbessertes Signal/Rausch-Verhältnis aus, ohne dass der apparative Aufwand erhöht wird.

Mit dem erfindungsgemäßen Verfahren lassen sich verschiedenste Eigenschaften von fließendem Blut im Innern eines Körpers in-vivo messen. Dabei kann es sich z. B. um die Bestimmung der Glukosekonzentration im fließenden Blut handeln. Dabei kann z. B. auf die Erkenntnisse aus der DE 10 2006 036 920 B3 oder der EP 1 601 285 B1 zurückgegriffen werden. Ebenso kommt die Bestimmung der Hämoglobinkonzentration oder der Sauerstoffsättigung des Blutes in Betracht (vgl. z.B. EP 1 601 285 B1). Alternativ oder ergänzend lässt sich mit dem erfindungsgemäßen Verfahren auch die Temperatur von fließendem Blut im Innern eines Körpers bestimmen. Dabei kann auf die Erkenntnisse aus der DE 10 2008 006 245 A1 zurückgegriffen werden. Unabhängig davon, welche Lichtwellenlängen verwendet werden und auf welche Weise aus den optischen Messungen dann die gewünschten Messwerte bestimmt werden, lässt sich erfindungsgemäß stets der relevante, aus dem fließenden Blut rückgestreute Lichtanteil in der beschriebenen Weise extrahieren, so dass die jeweilige Analyse optimiert wird.

Im Rahmen der Erfindung ist vorgesehen, mit nicht fokussierter Ultraschallstrahlung zu arbeiten, da auch in diesem Fall aus den erläuterten Gründen ein exaktes Extrahieren der relevanten Lichtanteile möglich wird. Bevorzugt wird der Körper mit gepulster Ultraschallstrahlung mit vorgegebener Pulslänge und Repititionszeit bestrahlt. Dabei wird die Lichtintensität an dem Detektor in einem um eine Verzögerung zeitlich verschobenen Zeitfenster gemessen. Dieses Zeitfenster entspricht der Pulslänge, wobei die Lichtintensität über dieses Zeitfenster integriert wird. Diese Vorgehensweise ermöglicht es, dass der Messbereich auf den relevanten Bereich reduziert und insbes. die Menge der aufgenommenen Daten deutlich reduziert wird, da die Messung auf solche Zeitfenster beschränkt wird, in denen der Ultraschallpuls das Blutgefäß erreicht.

Wie beim Stand der Technik ist es zweckmäßig, das Blutgefäß zunächst mit der Ultraschalleinrichtung im Vorfeld der Messung zu lokalisieren. Dazu werden einerseits eine Ultraschallquelle und andererseits ein Ultraschallempfänger verwendet, wobei der Ultraschallempfänger das Ultraschallecho auswertet. Aufgrund der (hörbaren) Dopplerverschiebung lässt sich bei der Abtastung der Tiefe die Blutbahn finden, so dass die Messung dann auf diesen Bereich konzentriert werden kann. Besonders bevorzugt wird man dabei in grundsätzlich bekannter Weise einen Ultraschalltransducer verwenden, der folglich zugleich Ultraschallquelle und Ultraschallempfänger ist.

Ferner ist es zur Optimierung der Auswertung zweckmäßig, eine Referenzmessung ohne Lichteinstrahlung durchzuführen und diese Referenzmessung im Zuge der Auswertung zu berücksichtigen.

Als Lichtquelle wird besonders bevorzugt zumindest ein Laser verwendet, der z. B. monochromes, kohärentes, kontinuierliches Laserlicht mit vorgegebener Wellenlänge erzeugt. Dabei werden grundsätzlich bekannte Wellenlängen verwendet, die für die jeweiligen optischen Messungen zweckmäßig sind und die aus dem Stand der Technik grundsätzlich bekannt ist. Dabei kann es für die jeweiligen Messungen auch zweckmäßig sein, mehrere unterschiedliche Wellenlängen einzustrahlen und ggf. mit mehreren Laserquellen zu arbeiten. Soll z. B. die Glukosekonzentration bestimmt werden, so bietet sich die Einstrahlung von zumindest zwei Wellenlängen aus dem Wellenlängenbereich 1560 bis 1630 nm einerseits und 790 bis 815 nm andererseits an (vgl. DE 10 2006 036 920 B3). Im Falle der Bestimmung der Hämoglobinkonzentration oder Sauerstoffsättigung kommen andere Wellenlängen in Betracht (vgl. EP 1 601 285 B1). Im Falle der Temperaturmessung kommen Wellenlängen im Bereich einer entsprechenden Absorptionslinie des Wassers in Betracht, wobei z. B. im Bereich der Wasserabsorptionslinie um 970 nm gearbeitet werden kann (vgl. DE 10 2008 006 245 1).

Im Folgenden wird die Erfindung anhand einer lediglich ein Ausführungsbeispiel darstellenden Zeichnung näher erläutert.

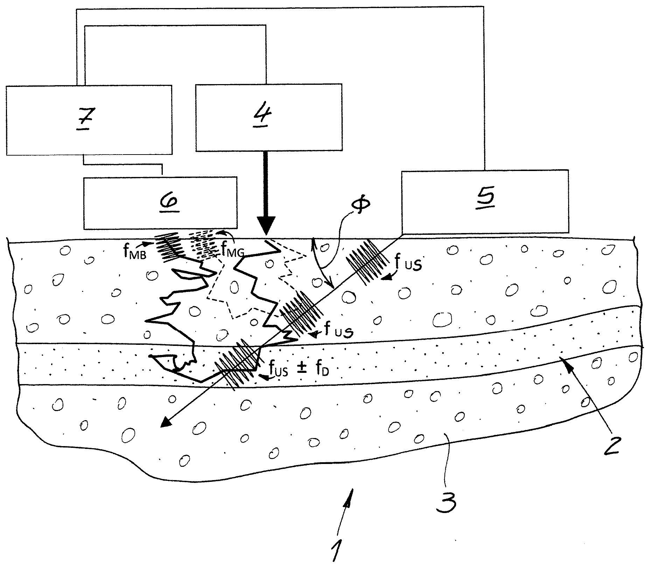

Die einzige Figur zeigt schematisch eine Vorrichtung zur Durchführung des beschriebenen Verfahrens.

In der Figur ist ein Körper 1 mit einem Blutgefäß 2 und dem an das Blutgefäß angrenzenden Gewebe 3 dargestellt. Zur nicht invasiven optischen Messung von Eigenschaften des Blutes sind eine Lasereinheit 4, eine Ultraschalleinheit 5, eine Detektoreinheit 6 und eine Steuerungs- und Auswerteeinheit 7 vorgesehen. Der Körper 1 mit dem Blutgefäß 2 wird mit der Lasereinheit 4 mit Licht mit zumindest einer Wellenlänge beleuchtet. Das rückgestreute Licht wird mit der Detektoreinheit 6 erfasst. Diese Detektoreinheit 6 misst lediglich Intensitäten, d. h., es erfolgt die Ermittlung des rückgestreuten Photonenstroms ohne Ortsauflösung oder Frequenzauflösung am Detektor. Die Wellenlänge des eingestrahlten Laserlichtes hängt von der Anwendung und folglich davon ab, welche Eigenschaften bzw. Bestandteile des Blutes analysiert werden sollen.

Erfindungsgemäß wird der Körper 1 zur Markierung des Blutgefäßes 2 mit Ultraschallstrahlung mit einer Ultraschallfrequenz fus bestrahlt. Aufgrund der Wechselwirkung der Ultraschallstrahlung mit dem Blut bzw. Gewebe erfolgt eine Modulation der rückgestreuten Lichtintensität mit der Frequenz der Ultraschallstrahlung. Von besonderer Bedeutung ist dabei die Tatsache, dass der außerhalb des Blutgefäßes 2, d.h. in dem angrenzenden Gewebe 3 rückgestreute Lichtanteil mit einer Frequenz $f_{MG}$ moduliert ist, die exakt der Ultraschallfrequenz fus entspricht. Demgegenüber ist der innerhalb des Blutgefäßes 2 rückgestreute Lichtanteil aufgrund des Dopplereffektes in fließendem Blut mit einer Frequenz $f_{MB}$ moduliert, welche gegenüber der Ultraschallfrequenz fus um die Dopplerverschiebung $f_D$ verschoben ist.

In der Figur ist daher angedeutet, dass die Detektoreinheit 6 sowohl Lichtanteile erreicht, die mit der Frequenz $f_{MB}$ moduliert sind als auch Lichtanteile, die mit der Frequenz $f_{MG}$ moduliert sind. Die mit der Frequenz $f_{MG}$ modulierten Lichtanteile gehen auf Streuungen im Gewebe 3 zurück, während die Lichtanteile mit der Frequenz $f_{MB}$ tatsächlich auf Streuungen innerhalb der Blutbahn 2 zurückgehen. Darüber hinaus erreichen die Detektoreinheit 6 aber auch Lichtanteile, die überhaupt nicht moduliert sind, da sie aus Bereichen stammen, die nicht mit einem Ultraschallpuls wechselwirken.

Erfindungsgemäß wird nur der Photonenstromanteil extrahiert, der mit der Frequenz $f_{MB}$ moduliert ist und folglich tatsächlich aus dem Bereich des bewegten Blutes rückgestreut wird. Es wird folglich die Dopplerverschiebung im optischen Signal analysiert. Der gesamte rückgestreute Photonenstrom besteht aus einer zeitlich konstanten Komponente und zwei modulierten Komponenten, nämlich zum einen der im Gewebe modulierten Komponente $f_{MG}$ und zum anderen der im Blut modulierten Komponenten mit der Frequenz $f_{MB}$. Ergänzend wird an dem Detektor auch ein Hintergrundrauschen erfasst, das unabhängig von dem eingestrahlten Licht ist.

Die Messung mit dem beschriebenen Verfahren kann z.B. wie folgt durchgeführt werden: Zunächst erfolgt die Suche eines Blutgefäßes. Dazu wird der gepulste Ultraschall in den Körper 1 oberhalb des Blutgefäßes 2 unter entsprechendem Winkel Φ eingestrahlt. Es erfolgt eine Abtastung der Tiefe axial mit ausgewählten Laufzeiten. Durch Analyse des Ultraschallechos lässt sich das Blutgefäß 2 lokalisieren. Das maximale Ultraschallecho entspricht der Laufzeit, bei der sich der Ultraschallpuls im Blutgefäß befindet. Die Laufzeit des maximalen Ultraschallechos entspricht der Hälfte der Zeit, die der Ultraschall für den Weg durch das Gewebe vom Ultraschallwandler zum Ultraschallempfänger benötigt. Das auf diese Weise ausgewertete Ultraschallecho erzeugt dann ein Signal, z. B. ein Audiosignal, ein Lichtsignal o. dgl.. Es wird dann ein Trigger-Signal auf eine Verzögerung (delay) eingestellt, wobei diese Verzögerung der Laufzeit des maximalen Ultraschallechos nach der Pulserzeugung entspricht. Dieses Trigger-Signal startet dann die folgende optische Messung.

Zur optischen Messung wird Laserlicht in den Körper 1 eingestrahlt. Die Erfassung der Detektordaten erfolgt in dem eingestellten Zeitfenster für das maximale Ultraschallechosignal. Auf diese Weise wird gewährleistet, dass der Zeitraum der Messung und damit auch die aufgenommenen Daten auf die Zeitbereiche beschränkt werden, in denen auch tatsächlich eine Modulation mit Hilfe eines Ultraschallpulses im Bereich der Blutbahn zu erwarten ist. Ein Messvorgang durch Aufnahme des von der Laserstrahlung rückgestreuten Lichtes besteht aus einer Folge von wiederholten optischen Aufnahmen mit dem Detektor in dem Zeitfenster. Auf diese Weise lassen sich die optischen Signale der niedrigen Frequenz - Dopplerverschiebung (hörbare Frequenzen im Bereich Hz, KHz) aus den optischen Signalen der Frequenz des Ultraschalls (Bereich MHz) extrahieren. Die Laserstrahlung wird während der Wiederholung innerhalb eines Messvorganges kontinuierlich eingestrahlt, d. h. der Laser bleibt während der Wiederholung eingeschaltet. Nach erfolgter Messung wird der Laser ausgeschaltet bzw. die Einstrahlung des Laserlichtes beendet.

Um das Hintergrundrauschen extrahieren zu können, wird der Messvorgang auch ohne Lasereinstrahlung wiederholt. Sofern für eine bestimmte Messung mit mehreren Wellenlängen gearbeitet wird und z. B. mehrere Laser verwendet werden, kann ggf. eine Wiederholung der einzelnen Schritte erfolgen.

Im Zuge der Auswertung wird nun berücksichtigt, dass das an dem Detektor eingehende Signal, d. h. der Photonenstrom neben dem laserunabhängigen Hintergrundrauschen einen nicht modulierten Anteil und folglich eine zeitlich konstante Komponente (DC-Wert) enthält. Außerdem enthält das Signal zwei modulierte Anteile und folglich zwei "AC-Komponenten". Der eine modulierte Anteil geht auf die Rückstreuung aus dem Gewebe zurück. Dieser Anteil ist mit der Frequenz $f_{MG}$

moduliert, welcher exakt der Frequenz der Ultraschallstrahlung fus entspricht. Dieser Anteil aus dem statischen Teil des Gewebes ist folglich periodisch mit der Ultraschalfrequenz fus in der Größenordnung Megahertz moduliert. Außerdem fällt ein zweiter modulierter Anteil auf den Detektor, der aufgrund der Dopplerverschiebung in dem fließenden Blut mit einer verschobenen Frequenz $f_{MB}$ moduliert ist. Diese Frequenz $f_{MB}$ weicht folglich um die Dopplerverschiebung $f_D$ von der Ultraschallfrequenz fus ab ($f_{MB}$ = fus $\pm$ $f_D$). Aufgrund des Pulsierens des Blutes wird eine Mischung von mehreren niedrigen Frequenzen in der Größenordnung Hertz und Kilohertz registriert. Auf diese Weise lässt sich der auf die Streuung in der Blutbahn zurückgehende Signalanteil extrahieren und daraus in der bekannten Weise die jeweilige Eigenschaft des Blutes, z. B. die Konzentration bestimmter Blutbestandteile oder auch die Temperatur bestimmten.

Die erfindungsgemäße Vorrichtung setzt sich folglich in grundsätzlich bekannter Weise aus einer Ultraschalleinheit 5, zumindest einer Lichtquelle 4, z. B. einer Laserlichtquelle und einer Detektoreinheit 6 sowie insbes. einer Steuerungs- und Auswerteeinheit 7 zusammen, wobei die Steuerungs- und Auswerteeinheit 7 in der erfindungsgemäßen Weise angepasst wird. Die Ultraschalleinheit 5 erzeugt die Ultraschallstrahlung, wobei diese nicht fokussiertist. Sie sendet ein gepulstes Signal aus. Neben einer Ultraschallquelle weist die Ultraschalleinheit 6 auch einen oder mehrere Empfänger auf, welcher die Signale, die im eingestellten Zeitfenster betrachtet werden, empfängt. Ultraschallsender und Ultraschallempfänger können auch in einem gemeinsamen Transducer zusammengefasst sein. Als Lichtquelle 4 wird bevorzugt eine Laserlichtquelle verwendet, die kontinuierliches, monochromatisches, kohärentes Licht der gewünschten Wellenlänge erzeugt. Es handelt sich folglich bevorzugt um einen CW-Laser.

Die Detektoreinheit 6 weist einen oder mehrere Detektoren auf, die seriell oder parallel miteinander verbunden werden und auf sehr einfache Weise das aus dem Körper austretende Licht detektieren. Dabei wird auf eine ortsaufgelöste Messung im Detektor und auch auf eine frequenzaufgelöste Messung verzichtet. Es erfolgt lediglich die Messung von Lichtintensitäten.

Die Steuerungs-Auswerteeinheit 7 steuert zunächst einmal die Ultraschalleinheit 5. Sie stellt das Zeitfenster ein, und erzeugt das Triggersignal für den Start und den Stopp der optischen Aufnahmen. Sie kann auch den Laser 4 ein- oder ausschalten bzw. die Lasereinstrahlung beginnen und beenden. Sie führt auch den Mess- und Auswertealgorithmus aus und sorgt für eine entsprechende Signalkonditionierung (Verstärkung, Filterung usw.). Mit Hilfe der Steuerungs-Auswerteeinheit 7 erfolgt dann auch eine Trennung der nicht modulierten und der modulierten Anteile aus dem Detektorsignal. Dabei kann auf grundsätzlich bekannte klassische Methoden zur Isolierung niedriger Frequenzen aus hochfrequenten Mischsignalen, z.B. eine Fourier-Analyse, zurückgegriffen werden.

**Patentansprüche**

1. Verfahren zur nichtinvasiven optischen in-vivo-Messung von Eigenschaften von fließendem Blut in einem Blutgefäß im Innern eines Körpers, z. B. zur Bestimmung der Konzentration von Blutbestandteilen,

   wobei der Körper zur Markierung eines Blutgefäßes mit nicht fokussierter Ultraschallstrahlung mit einer Ultraschallfrequenz (fus) bestrahlt wird,
   wobei der Körper mit dem Blutgefäß mit Licht mit zumindest einer Lichtwellenlänge beleuchtet und das rückgestreute Licht mit einem Detektor erfasst wird,
   wobei der außerhalb des Blutgefäßes aus dem Körper rückgestreute Lichtanteil mit einer Frequenz ($f_{MG}$) moduliert ist, welche der Frequenz (fus) der Ultraschallstrahlung entspricht,
   wobei der innerhalb des Blutgefäßes rückgestreute Lichtanteil aufgrund des Dopplereffektes in fließendem Blut mit einer um die Dopplerverschiebung ($f_D$) gegenüber der Frequenz (fus) der Ultraschallstrahlung verschobenen Frequenz ($f_{MB}$) moduliert ist und
   wobei mit einer Auswerteeinheit aus dem an dem Detektor gemessenen Detektorsignal nur der mit der verschobenen Frequenz ($f_{MB}$) modulierte Signalanteil extrahiert wird.

2. Verfahren nach Anspruch 1, wobei der Körper mit gepulster Ultraschallstrahlung mit vorgegebener Pulslänge und Repititionszeit bestrahlt wird und wobei die Lichtintensität an dem Detektor in einem um eine Verzögerung zeitlich verschobenen Zeitfenster, welches der Pulslänge der Ultraschallstrahlung entspricht, gemessen wird.

3. Verfahren nach Anspruch 1 oder 2, wobei das Blutgefäß vor der optischen Messung zunächst durch (akustische) Analyse des aus dem Körper rückgestrahlten Ultraschallechos lokalisiert wird.

4. Verfahren nach einem der Ansprüche 1 bis 3, wobei eine Referenzmessung ohne Lichteinstrahlung durchgeführt wird und wobei die Referenzmessung bei der Auswertung berücksichtigt, wird.

5. Verfahren nach einem der Ansprüche 1 bis 4, wobei das Licht mit zumindest einer Laserlichtquelle erzeugt wird.

6. Verfahren nach einem der Ansprüche 1 bis 5, wobei Licht mehrerer unterschiedlicher Lichtwellenlängen eingestrahlt wird, z.B. mit mehreren Laserlichtquellen, wobei die Einstrahlung und Messung zeitlich nacheinander oder gleichzeitig erfolgt.

**Claims**

1. Method for non-invasive optical *in-vivo* measurement of properties of flowing blood in a blood vessel within a body, for determining the concentration of blood constituents, for example,

   wherein the body is irradiated with unfocused ultrasonic radiation having an ultrasonic frequency ($f_{US}$) in order to mark a blood vessel, wherein the body with the blood vessel is illuminated with light having at least one light wavelength, and the backscattered light is detected with a detector,
   wherein the light component outside the blood vessel that is backscattered out of the body is modulated with a frequency ($f_{MG}$) that corresponds to the frequency ($f_{US}$) of the ultrasonic radiation,
   wherein the light component that is backscattered inside the blood vessel is modulated with a frequency ($f_{MB}$) that is shifted by the Doppler shift ($f_D$) with respect to the frequency ($f_{US}$) of the ultrasonic radiation due to the Doppler effect in flowing blood, and
   wherein only the signal component that has been modulated with the shifted frequency ($f_{MB}$) is extracted with an evaluation unit from the detector signal measured at the detector.

2. Method according to Claim 1, wherein the body is irradiated with pulsed ultrasonic radiation having a predetermined pulse length and repetition time, and wherein the light intensity is measured at the detector in a time window shifted by a delay time, which window corresponds to the pulse length of the ultrasonic radiation.

3. Method according to Claim 1 or 2, wherein the blood vessel is first located before the optical measurement by (acoustic) analysis of the ultrasonic echo backscattered from the body.

4. Method according to any one of Claims 1 to 3, wherein a reference measurement is performed without a light penetration, and wherein the reference measurement is taken into account in the analysis.

5. Method according to any one of Claims 1 to 4, wherein the light is generated using at least one laser light source.

6. Method according to any one of Claims 1 to 5, wherein light is introduced with a plurality of different wavelengths, generated for example by a plurality of light sources, wherein penetration and measurement are carried out one after the other or simultaneously.

**Revendications**

1. Procédé de mesure optique in-vivo non invasive de propriétés de sang coulant dans un vaisseau sanguin à l'intérieur d'un corps, par exemple pour déterminer la concentration de composantes du sang,

   dans lequel le corps est irradié avec une fréquence d'ultrasons ($f_{US}$) pour marquer un vaisseau sanguin avec un rayonnement par ultrasons non focalisé,
   dans lequel le corps avec le vaisseau sanguin est éclairé avec de la lumière avec au moins une longueur d'onde lumineuse et la lumière rétro-diffusée est détectée avec un détecteur,
   dans lequel la part de lumière rétro-diffusée du corps hors du vaisseau sanguin est modulée à une fréquence ($f_{MG}$) qui correspond à la fréquence ($f_{US}$) du rayonnement par ultrasons,
   dans lequel la part de lumière rétro-diffusée à l'intérieur du vaisseau sanguin, en raison de l'effet de doppler dans le sang qui coule, est modulée avec une fréquence décalée ($f_{MB}$) du décalage de doppler ($f_D$) par rapport à la fréquence ($f_{US}$) du rayonnement par ultrasons et
   dans lequel avec une unité d'évaluation, seule la part du signal modulée avec la fréquence décalée ($f_{MB}$) est extraite du signal de détecteur mesuré sur le détecteur.

2. Procédé selon la revendication 1, dans lequel le corps est irradié avec un rayonnement par ultrasons pulsé avec une longueur d'impulsion et un temps de répétition prédéfinis et dans lequel l'intensité de lumière est mesurée sur le détecteur, dans une fenêtre temporelle décalée temporellement d'une temporisation qui correspond à la longueur d'onde du rayonnement par ultrasons.

3. Procédé selon la revendication 1 ou 2, dans lequel le vaisseau sanguin est localisé avant la mesure optique d'abord par analyse (acoustique) de l'écho d'ultrasons rétro-diffusé du corps.

4. Procédé selon l'une des revendications 1 à 3, dans lequel une mesure de référence est effectuée sans rayonnement de lumière, et dans lequel la mesure de référence est prise en compte lors de l'évaluation.

5. Procédé selon l'une des revendications 1 à 4, dans lequel la lumière est produite avec au moins une source de lumière laser.

6. Procédé selon l'une des revendications 1 à 5, dans lequel de la lumière de plusieurs longueurs d'onde différentes est irradiée, par exemple avec plusieurs sources de lumière laser, dans lequel le rayonnement et la mesure ont lieu de manière consécutive

temporellement ou simultanément.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1601285 B1
- DE 102006036920 B3
- DE 102008006245 A1
- US 20060253007 A1
- DE 1020080062451